# EUROPEAN PATENT APPLICATION

(11) **EP 2 279 728 A1**
(43) Date of publication of application: **02.02.2011**
(21) Application number: 10171550.6
(22) Date of filing: 02.08.2010
(51) Int. Cl.: A61K 9/14, A61K 9/16, A61K 9/20

(54) **Solid dosage forms of HIV protease inhibitors**

(30) Priority: 31.07.2009 IN DE15962009
(71) Applicant: Ranbaxy Laboratories Limited, Gurgaon - 122001, Haryana (IN)
(72) Inventor: Tiwari, Ravindra, 324002, Rajasthan (IN); Janakiraman, Kannayiram K., 608001, Tamil Nadu (IN); Agarwal, Ravindra, 313002, Rajasthan (IN); Raghuvanshi, Rajeev Singh, 122001, Haryana (IN)
(74) Representative: Cronin, Brian Harold John

(57) **Abstract**

The present invention relates to a solid dosage form comprising a solid dispersion composition of at least one HIV protease inhibitor and water soluble polymer having a glass transition temperature (Tg) of at least about 50°C in an amount of less than 50% by weight of the dosage form. It also relates to a process of preparation of such solid dosage forms.

## Description

### Field of the Invention

The present invention relates to a solid dosage form which includes a solid dispersion composition of at least one HIV protease inhibitor and water soluble polymer having a glass transition temperature (Tg) of at least about 50°C in an amount of less than 50% by weight of the said dosage form. It also relates to a process of preparation of such solid dosage forms.

### Background of the Invention

Human immuno-deficiency virus (HIV), the causative agent for AIDS, belongs to a class of viruses known as retroviruses, which carry genetic information in the form of RNA. As the virus enters the host cell, its RNA is reverse-transcribed to DNA by a virally encoded enzyme, reverse transcriptase. The viral DNA enters the host cell nucleus, where it is integrated into the genetic material of the cell by a second virally encoded enzyme called integrase. Activation of the host cell results in the transcription of the viral DNA to messenger RNA, which is then translated onto viral proteins. HIV protease, a 99-amino acid homodimer, is the enzyme which mediates this step of viral maturation by cleaving the viral gag-pol polyprotein precursor into essential functional proteins. In the event that these polyproteins are not cleaved, the virus fails to mature and is incapable of infecting a new cell.

The HIV protease inhibitors are a class of antiretroviral agents that competitively compete for this HIV proteinase or protease enzyme. These are peptide like molecules that mimic the gag-pol protein, binding onto HIV proteases to prevent the accumulation of structural proteins required for new virion formation. The HIV protease inhibitors have contributed greatly to the reductions in HIV-associated morbidity and mortality over the last decade and remain a cornerstone of highly-active antiretroviral therapy (HAART).

Ritonavir is one of the prominent members of this class of compounds, which is commercialized as Norvir^{®} oral solution and soft gelatin capsules by Abbott Laboratories, USA. U.S. Patent Nos. 5,542,206 and 5,648,497 disclose ritonavir and describes its use as an inhibitor of the HIV protease enzyme. Additionally, ritonavir is used extensively as a pharmacokinetic enhancer when co-administered with other protease inhibitors. U.S. Patent Nos. 6,037,157 and 6,703,403 disclose the use of ritonavir in combination with an HIV protease inhibitor, which is metabolized by cytochrome P450 monooxygenase, such that the amount of ritonavir is sufficient to improve the pharmacokinetics of the HIV protease inhibitor in a patient, relative to the pharmacokinetics of the HIV protease inhibitor when administered alone. Ritonavir is dosed as a pharmacokinetic enhancer with amprenavir, atazanavir, fosamprenavir, lopinavir, saquinavir, tipranavir, darunavir, and the like.

Ritonavir is also available as a co-formulated composition with lopinavir, another potent HIV protease inhibitor, under the proprietary names Kaletra^{®}/Aluvia^{®} as soft gel capsules and tablets from Abbott Laboratories, USA. Lopinavir is described specifically in U.S. Patent No. 5,914,332.

Despite tremendous success worldwide, the main drawback that remains associated with HIV protease inhibitors is that they exhibit low oral bioavailability. A majority of such HIV protease inhibitors, and ritonavir specifically, pose great difficulty in regards to formulating into an appropriate, patient-compliant dosage forms, owing to the fact that these are BCS class II and IV active ingredients. Formulating the protease inhibitors in liquid dosage forms is not always a preferable solution to improve upon the problem of bioavailability, namely due to unpleasant taste, stability problems, handling and storage hazards, patient incompliance, etc.

One solution to provide a solid unit dosage composition of such drugs is to formulate a solid dispersion composition. A "solid dispersion" of a poorly soluble active ingredient, can be defined as an even dispersion of the active ingredient in an inert carrier in solid state. The active ingredient is dispersed in the carrier by coprecipitation from a suitable solution containing both the active ingredient and carrier, by melting both components together or by some other process involving a phase change. The use of a surfactant in such solid dispersion compositions to form a ternary system is also well documented.

Consequently, attempts have been made in the art to formulate solid dosage forms that improve the bioavailability of solid oral dosage forms comprising HIV protease inhibitors by preparing solid dispersion compositions. The PCT Publication WO 2005/039551 teaches one such formulation exhibiting improved bioavailability, which comprises of a solid dispersion comprising at least one HIV protease inhibitor and at least one pharmaceutically acceptable water-soluble polymer having a Tg of at least about 50°C and at least one pharmaceutically acceptable surfactant, wherein the amount of polymer is at least 50% by weight of the total weight of the formulation. Another PCT Publication WO 2006/091529 discloses similar formulations which do not exhibit any food effects.

The present invention relates to a solid dosage form comprising a solid dispersion composition of at least one HIV protease inhibitor and water soluble polymer having a glass transition temperature (Tg) of at least about 50°C in an amount of less than 50% by weight of the said dosage form.

### Summary of the Invention

In one general aspect, the present invention provides for a solid dosage form that includes a solid dispersion composition. The solid dispersion includes the following:
(a) at least one HIV protease inhibitor;
(b) a water soluble polymer having a glass transition temperature (Tg) of at least about 50 °C in an amount of less than 50% by weight of the solid dosage form;
(c) a pharmaceutically acceptable surfactant;
(d) optionally, a pharmaceutically acceptable carrier; and
(e) optionally, other pharmaceutically acceptable excipients.

Embodiments of the invention may include one or more of the following features. For example, the HIV protease inhibitor(s) utilized is ritonavir, lopinavir, atazanavir, amprenavir, fosamprenavir, saquinavir, tipranavir, or darunavir, or combinations thereof. Preferably, the HIV protease inhibitor is ritonavir or ritonavir and lopinavir.

The solid dispersion may include two HIV protease inhibitors in a ratio of about 1:15 to about 15:1 by weight. For example, the lopinavir and ritonavir is in a ratio of 1:4. The water soluble polymer is present in an amount from about 35% to about 48% by weight of the solid dosage form. For example, the water soluble polymer is a copolymer of N-vinyl pyrrolidone and vinyl acetate.

Suitable pharmaceutically acceptable surfactants include polyoxyethylene castor oil derivates, sorbitan monolaurate, stearoyl macrogolglycerides or combinations thereof.

The pharmaceutically acceptable surfactant is present in an amount of about 1% to about 20% by weight of the solid dosage form.

The pharmaceutically acceptable carrier has a glass transition temperature (Tg) of less than 50°C. For example, suitable pharmaceutically acceptable carriers include polyethylene glycol or polyethylene glycol 6000. The pharmaceutically acceptable carrier is present in an amount from 0% to about 30% by weight of the solid dosage form.

In another general aspect, the present invention provides a process for preparing a solid dosage form. The process includes the following steps:
(a) blending at least one HIV protease inhibitor with a water soluble polymer having a glass transition temperature (Tg) of at least about 50°C, optionally, a pharmaceutically acceptable carrier and optionally, an additional other pharmaceutically acceptable excipients in a suitable mixer;
(b) mixing the blend of step (a with a pharmaceutically acceptable surfactant and further blending;
(c) transferring the blend of step (b) to a suitable melt-extruder and melting at appropriate temperature to form a molten extrudate mass;
(d) cooling the molten extrudate mass of step (c), and sizing to obtain a solid dispersion;
(e) blending the solid dispersion of step (d) with other one or more pharmaceutically acceptable excipient(s) in a suitable blender to obtain the final blend; and
(f) processing the final blend of step (e) into a solid dosage form using appropriate tooling.

### Detailed Description of the Invention

The term "solid dosage form", as used herein, encompasses all standard pharmaceutical solid dosage forms and may be in the form of coated or uncoated tablets, multilayer tablets, hard gelatin capsules, soft gelatin capsules, pills and the like. The "solid dosage form" may also be in granular or powder forms which are processed in sachets, pouches, packages, and the like.

The solid dosage form as described herein comprises of a solid dispersion composition. The term solid dispersion refers to a group of solid products that include a pharmaceutically acceptable matrix and at least one HIV protease inhibitor drug homogeneously dispersed therein. The matrix may be either crystalline or amorphous. The drug may be dispersed molecularly, in amorphous particles (clusters) or in crystalline particles.

The "HIV protease inhibitor", as described herein, includes an effective amount of the compound, pharmaceutically acceptable salts, solvates, enantiomers, diastereomers or polymorphs thereof. The suitable "HIV protease inhibitor", for use herein, includes ritonavir; lopinavir; indinavir; saquinavir; amprenavir; fosamprenavir; mozenavir; nelfinavir; atazanavir ; tipranavir; palinavir; darunavir; Ro033-4649; DMP-323; 5(S)-Boc-amino-4(S)-hydroxy-6-phenyl-2(R)-phenyl methyl hexanoyl-(L)-V-al-(L)-Phe-morpholin-4-ylamide; [1-naphthoxyacetyl-beta-methylthio-Ala-(2S,3S)3-amino-2-hydroxy-4-butanoyl-1,3-thiazolidine-4t-butylamide; 5 - isoquinolinoxyacetyl-beta-methylthio - Ala - (2S,3S)-3amino-2-hydroxy-4-butanoyl-1,3-thiazolidine-4-tbutylamide; [1S-[[1R-(R-),2S*])-N'[3-[[[(1,1-dimethyl ethyl)amino]carbonyl](-2-methyl propyl)amino]-2hydroxy-1-(phenyl methyl) propyl]-2-[(2-quinolinyl carbonyl)amino]-butanediamide; P-1946; BMS 186,318; SC-55389a; BILA 1096 BS; U-140690, or combinations thereof.

The term "effective amount" in the present invention refers to the HIV protease inhibitor in amounts suitable to elicit a particular biological or medicinal or clinical response being sought by the person skilled in the art. The effective amount further refers to a "therapeutically effective amount" which results in the alleviation of the symptoms of the disease or condition being treated by the drug. The effective amount also refers to a "prophylactically effective amount" which results in prophylaxis of the symptoms of the disease or condition being prevented by the drug. The effective amount also refers to an amount that would provide enhanced therapeutic activity of another drug that is co-administered with it, in a way that if the later drug was administered alone, would not have achieved the desired response (*e.g.*, unsatisfactory pharmacokinetic values for the drug and/or an unsatisfactory drug circulation level resulting in little or no efficacy).

In one embodiment, the "HIV protease inhibitor" is ritonavir, and may include one or more additional HIV protease inhibitors, including lopinavir, atazanavir, amprenavir, fosamprenavir, saquinavir, tipranavir, and darunavir.

The amount of HIV protease inhibitor may be in an amount ranging from 1mg to 1500mg. When two HIV protease inhibitors are present, it may be present in a ratio of about 1:15 to about 15:1 by weight, particularly in a ratio of about 1:10 to about 10:1 1 by weight, more particularly in a ratio of about 1:4 to about 4:1 by weight.

The term "water soluble polymer having a glass transition temperature (Tg) of at least about 50°C", as referred to herein, allows for the preparation of solid dispersions that are mechanically stable and, within ordinary temperature ranges, sufficiently temperature stable.

Such water-soluble polymers include homopolymers and copolymers of N-vinyl lactams, especially homopolymers and copolymers of N-vinyl pyrrolidone, e. g., polyvinylpyrrolidone (PVP), copolymers of N-vinyl pyrrolidone and vinyl acetate or vinyl propionate, cellulose esters and cellulose ethers, such as methylcellulose and ethylcellulose, hydroxyalkylcelluloses, in particular hydroxypropylcellulose, hydroxyalkylalkylcelluloses, in particular hydroxypropylmethylcellulose, cellulose phthalates or succinates, in particular cellulose acetate phthalate and hydroxypropylmethylcellulose phthalate, hydroxypropylmethylcellulose succinate or hydroxypropylmethylcellulose acetate succinate; high molecular polyalkylene oxides such as polyethylene oxide and polypropylene oxide and copolymers of ethylene oxide and propylene oxide, polyacrylates and polymethacrylates such as methacrylic acid/ethyl acrylate copolymers, methacrylic acid/methyl methacrylate copolymers, butyl methacrylate/2- dimethylaminoethyl methacrylate copolymers, poly (hydroxyalkyl acrylates), poly (hydroxyalkyl methacrylates), polyacrylamides, vinyl acetate polymers such as copolymers of vinyl acetate and crotonic acid, partially hydrolyzed polyvinyl acetate (also referred to as partially saponified "polyvinyl alcohol"), polyvinyl alcohol, oligo-and polysaccharides such as carrageenans, galactomannans and xanthan gum, or mixtures of one or more thereof. In one embodiment, copolymer of N-vinyl-2-pyrrolidone and vinyl acetate (like copovidone) such as those which are available as Plasdone^{®} or Kollidon^{®} from ISP and BASF respectively may be used. In one embodiment, the combination of polyethylene glycol and copolymer of N-vinyl-2-pyrrolidone and vinyl acetate (like copovidone) is used.

The amount of water soluble polymer having a glass transition temperature (Tg) of at least about 50°C, is in an amount less than 50% by weight of the solid dosage form, preferably in an amount of about 35% to about 48% by weight of the solid dosage form, and more preferably in an amount of about 40% to about 46% by weight of the solid dosage form.

The "pharmaceutically acceptable carrier", as described herein, refers to other polymeric and non-polymeric carrier substances in addition to the water-soluble polymer having a glass transition temperature (Tg) of at least about 50°C. The "pharmaceutically acceptable carrier" may be hydrophilic or hydrophobic in nature, and aid in forming stable solid dispersion compositions. Such "pharmaceutically acceptable carrier" may be exemplified as different grades of polyethylene glycol, Eudragit®EPO, and the like thereof. Such "pharmaceutically acceptable carrier" may have glass transition temperature (Tg) less than 50°C. In one embodiment, PEG 6000 is used as a pharmaceutically acceptable carrier.

The pharmaceutically acceptable carrier may be in an amount of 0% to about 30% of the total weight of the solid dosage form, preferably in an amount of about 5% to about 20% of the total weight of the solid dosage form.

The term "pharmaceutically acceptable surfactant", as described herein, includes polyoxyethylene alkyl ethers, *e.g.*, polyoxyethylene lauryl ether, polyoxyethylene cetyl ether, polyoxyethylene stearyl ethers; polyoxyethylene alkylaryl ethers, *e.g.*, polyoxyethylene nonylphenyl ethers, polyoxyethylene octylphenyl ethers; polyethylene glycol fatty acid esters, *e.g.*, PEG-200 monolaurate, PEG-200 dilaurate, PEG-300 dilaurate, PEG-400 dilaurate, PEG-300 distearate, PEG-300 dioleate; alkylene glycol fatty acid mono esters, *e.g.*, propylene glycol monolaurate; sucrose fatty acid esters, *e.g.,* sucrose monostearate, sucrose distearate, sucrose monolaurate, sucrose dilaurate; or sorbitan fatty acid mono esters such as sorbitan monolaurate (Span 20), sorbitan monooleate, sorbitan monopalmitate (Span 40), or sorbitan stearate, polyoxyethylene castor oil derivates, *e.g.*, polyoxyethyleneglycerol triricinoleate or (Cremophor^{®}EL; BASF Corp.) or polyoxyethyleneglycerol oxystearate such as polyethylenglycol 40 hydrogenated castor oil (Cremophor^{®}RH 40) or polyethylenglycol 60 hydrogenated castor oil (Cremophor^{®}RH 60); or block copolymers of ethylene oxide and propylene oxide, also known as polyoxyethylene polyoxypropylene block copolymers or polyoxyethylene polypropyleneglycol, such as Poloxamer^{®}124, Poloxamer^{®}188, Poloxamer^{®}237, Poloxamer^{®}388, Poloxamer^{®}407 (BASF Wyandotte Corp.); polyglycolized glycerides, for example, lauroyl macrogolglycerides (Gelucire^{®}44/14), stearoyl macrogolglycerides (Gelucire^{®}50/13); Labrasol^{®}, Transcutol^{®} (Gattefossé Canada Inc.); Vitamin E/TPGS: Tocopheryl propylene glycol 1000 succinate, sold by Eastman; polyethylene glycol 15 hydroxystearate (Solutol^{®}HS15 sold by BASF); or a mono fatty acid ester of polyoxyethylene (20) sorbitan, e. g., polyoxyethylene (20) sorbitan monooleate (Tween^{®} 80), polyoxyethylene (20) sorbitan monostearate (Tween^{®}), polyoxyethylene (20) sorbitan monopalmitate (Tween^{®}40), polyoxyethylene (20) sorbitan monolaurate (Tween^{®}20) or mixtures of one or more thereof. In one embodiment, the pharmaceutically acceptable surfactant is sorbitan monolaurate. The amount of pharmaceutically acceptable surfactant may vary from about 1% to about 20% by weight of the solid dosage form.

The solid dispersion composition may include one or more of pharmaceutically acceptable excipients, for example, lubricants and/or glidants which includes colloidal silica, stearic acid, magnesium stearate, calcium stearate, sodium stearyl fumarate, talc, hydrogenated vegetable oils, wax and the combinations thereof.

One or more of additional pharmaceutically acceptable excipients include fillers, disintegrants, glidants, lubricants, and combinations thereof, may be used in the solid dosage form as described herein.

Fillers may be selected from saccharides such as lactose, dextrose, sucrose, fructose, maltose; sugars such as mannitol, erythritol, sorbitol, xylitol and lactitol; cellulose derivatives such as powdered cellulose, microcrystalline cellulose; dicalcium phosphate, tribasic calcium phosphate, calcium sulphate, calcium carbonate, kaolin, starch and starch derivatives such as pregelatinized starch, partially pregelatinized starch; cellulose ethers such as carboxymethyl cellulose, methylcellulose, hydroxypropyl cellulose, hydroxypropyl methylcellulose; carboxy vinyl polymers such as carbomers; acrylates such as Eudragits; polyvinylpyrrolidone; xanthan gum, guar gum and other such materials routinely used in the art of solid dosage form manufacturing.

Disintegrants may include croscarmellose sodium, sodium starch glycolate, crosslinked polyvinylpyrrolidone, corn starch, potato starch, pregelatinized starch, low-substituted hydroxypropylcellulose, alginates, carboxymethyl starches, methacrylic acid divinylbenzene copolymer salts and microcrystalline cellulose.

Lubricants and/or glidants that may be used include magnesium stearate, calcium stearate, zinc stearate, sodium stearyl fumarate, powdered stearic acid, magnesium oleate, calcium palmitate, potassium laureate, sodium suberate, vegetable oil, mineral oil, talc, colloidal silicon dioxide, corn starch, and the like.

One or more of solvents may be used to dissolve and/or disperse one or more of the HIV protease inhibitor(s) and the additives, in a way that it does not create any stability issues and/or environmental hazards. Such solvents include one or more of alcohols, such as isopropyl alcohol or aliphatic hydrocarbons, such as acetone and esters.

In one embodiment, a solid dosage form includes a solid dispersion of ritonavir, a water soluble polymer having a glass transition temperature (Tg) of at least about 50°C, a pharmaceutically acceptable carrier, a pharmaceutically acceptable surfactant, a filler, a disintegrant, a glidant and a lubricant, wherein the polymer is in an amount of about 45% by weight of the solid dosage form.

In another embodiment, a solid dosage form includes a solid dispersion of ritonavir, another HIV protease inhibitor, a water soluble polymer having a glass transition temperature (Tg) of at least about 50°C, a pharmaceutically acceptable carrier, a pharmaceutically acceptable surfactant, a filler, a disintegrant, a glidant and a lubricant, wherein the polymer is in an amount of about 45% by weight of the solid dosage form.

In another embodiment, a solid dosage form includes a solid dispersion of ritonavir, lopinavir, a water soluble polymer having a glass transition temperature (Tg) of at least about 50°C, a pharmaceutically acceptable carrier, a pharmaceutically acceptable surfactant, a filler, a disintegrant, a glidant and a lubricant, wherein the polymer is in an amount of about 45% by weight of the solid dosage form and wherein the ritonavir and lopinavir are present in a ratio of 1:4 by weight.

In one aspect of the above embodiments, the water soluble polymer having a glass transition temperature (Tg) of at least about 50°C is a copolymer of N-vinyl-2-pyrrolidone and vinyl acetate (copovidone). In addition, in one aspect of the above embodiments, the pharmaceutically acceptable carrier is polyethylene glycol.

The solid dosage form may be prepared by employing conventional techniques known in the art, including dry granulation, aqueous and non-aqueous granulation, direct compression and roller compactation, melt granulation, melt extrusion, pelletization, solvent evaporation, and the combinations thereof.

In one embodiment the solid dosage form is prepared by the following steps:
1. blending an effective amount of ritonavir with a water soluble polymer having a glass transition temperature (Tg) of at least about 50°C glidant(s) and lubricant(s) in a suitable mixer;
2. mixing the blend of step (1) with the pharmaceutically acceptable surfactant and further blended;
3. transferring the blend of step (2) to a suitable melt-extruder and molten at appropriate temperature to form a molten extrudate mass;
4. cooling the molten extrudate mass of step (3) and sizing to obtain a solid dispersion;
5. blending the solid dispersion of step (4) with filler, disintegrant, glidant and lubricant in a suitable blender to obtain the final blend;
6. processing the final blend of step (5) into a solid dosage form using appropriate tooling.

In another embodiment the solid dosage form is prepared by the following steps:
1. blending an effective amount of ritonavir and another HIV protease inhibitor is blended with a water soluble polymer having a glass transition temperature (Tg) of at least about 50°C, glidant(s) and lubricant(s) in a suitable mixer;
2. mixing the blend of step (1) with a pharmaceutically acceptable surfactant and further blending;
3. transferring the blend of step (2) to a suitable melt-extruder and molten at appropriate temperature to form a molten extrudate mass;
4. cooling the molten extrudate mass of step (3) and sizing to obtain a solid dispersion;
5. blending the solid dispersion of step (4) with filler, disintegrant, glidant and lubricant in a suitable blender to obtain the final blend;
6. processing the final blend of step (5) into solid dosage form using appropriate tooling.

In another embodiment the solid dosage form is prepared by the following steps:
1. blending an effective amount of ritonavir with a water soluble polymer having a glass transition temperature (Tg) of at least about 50°C, a pharmaceutically acceptable carrier, glidant(s) and lubricant(s) in a suitable mixer;
2. mixing the blend of step (1) with a pharmaceutically acceptable surfactant and further blending;
3. transferring the blend of step (2) in a suitable melt-extruder and molten at appropriate temperature to form a molten extrudate mass;
4. cooling the molten extrudate mass of step (3) and sizing to obtain a solid dispersion;
5. blending the solid dispersion of step (4) with filler, disintegrant, glidant and lubricant in a suitable blender to obtain the final blend;
6. processing the final blend of step (5) into a solid dosage form using appropriate tooling.

In yet another embodiment the solid dosage form is prepared by the following steps:
1. blending the effective amount of ritonavir and another HIV protease inhibitor with a water soluble polymer having a glass transition temperature (Tg) of at least about 50°C, a pharmaceutically acceptable carrier, glidant(s) and lubricant(s) in a suitable mixer;
2. mixing the blend of step (1) with the pharmaceutically acceptable surfactant and further blending;
3. transferring the blend of step (2) to a suitable melt-extruder and molten at appropriate temperature to form a molten extrudate mass;
4. cooling the molten extrudate mass of step (3) and sizing to obtain a solid dispersion;
5. blending the solid dispersion of step (4) with filler, disintegrant, glidant and lubricant in a suitable blender to obtain the final blend;
6. processing the final blend of step (5) into a solid dosage form using appropriate tooling.

In one aspect of the above embodiment, the other HIV protease inhibitor is lopinavir.

The solid dosage form may further include a non-functional coating. Proprietary non-functional coating materials, such as Opaspray® and Opadry®), obtainable from Colorcon Ltd., UK, may be used.

From the above, it is apparent that various modifications and combinations of the formulations detailed in the text may be made without departing from the spirit and scope of the invention. The invention as described herein may be illustrated by the following examples but is not to be construed to be limiting by them.

### Examples 1 - 5

| **S/N** | **Ingredients** | **Quantity in mg/tablet** | | | | |
|---|---|---|---|---|---|---|
| | | **Example 1** | **Example 2** | **Example 3** | **Example 4** | **Example 5** |
| **Part (a) (Solid Dispersion)** | | | | | | |
| 1 | Ritonavir | 50.428 | 50.428 | 50.428 | 50.428 | 50.428 |
| 2 | Lopinavir | 201.509 | 201.509 | 201.509 | 201.509 | 201.509 |
| 3 | Copovidone | 553.000 | 553.000 | 553.000 | 553.000 | 553.000 |
| 4 | Polyethylene Glycol (PEG 6000) | - | - | - | 299.762 | - |
| 5 | Cremophor^{®}RH 40 | - | 60.000 | - | - | - |
| 6 | Sorbitan Monolaurate | 83.000 | 83.000 | 83.000 | 83.000 | 83.000 |
| 7 | Gelucire^{®}50/13 | - | - | 60.000 | - | - |
| 8 | Eudragit®EPO (Powder) | - | - | - | - | 230.000 |
| 9 | Colloidal Silicon Dioxide | 12.200 | 12.200 | 12.200 | - | 12.200 |
| 10 | Stearic Acid | - | - | - | - | 46.000 |

| **Part (b)** | | | | | | |
|---|---|---|---|---|---|---|
| 11 | Lactose | 299.762 | 239.762 | 239.762 | - | 35.762 |
| 12 | Colloidal Silicon Dioxide | 8.100 | 8.100 | 8.100 | 8.100 | 8.100 |
| 13 | Sodium Stearyl Fumarate | 12.000 | 12.000 | 12.000 | 12.000 | 12.000 |
| **Total Weight** | | ∼ **1220** | **∼ 1220** | ∼**1220** | ∼**1220** | ∼**1232** |

### General Procedure for Preparation of Examples 1 - 5

### Part (a):

The effective amounts of ritonavir and lopinavir is blended with colloidal silicon dioxide and either of copovidone (for Example 1, Example 2 and Example 3) or a combination of copovidone and polyethylene glycol (for Example 4), or a combination of copovidone and Eudragit®EPO (for Example 5) in a suitable mixer. To the blend, sorbitan monolaurate is added and further blended. Additionally, in Example 2 and Example 3, respectively, Cremophor^{®}RH 40 and Gelucire^{®}50/13 are also blended along with sorbitan monolaurate. The blend obtained is melted and the molten mass is subjected to extrusion at a temperature of up to about 130°C in a suitable Melt Extruder. The molten extrudate mass is cooled and is suitably sized to obtain a solid dispersion.

### Part (b):

The excipients of Part (b) are blended to form a homogeneous blend.

Part (a) and Part(b) are admixed and compressed to tablets using appropriate tooling.

### Examples 6 - 13

| **S. No** | **Ingredients** | **Quantity in mg/tablet** | | | | | | | |
|---|---|---|---|---|---|---|---|---|---|
| | | **Example 6** | **Example 7** | **Example 8** | **Example 9** | **Example 10** | **Example 11** | **Example 12** | **Example 13** |
| **Part (a) (Solid Dispersion)** | | | | | | | | | |
| 1. | Lopinavir | 100.00 | 200.00 | 100.00 | 200.00 | 100.00 | 200.00 | 100.00 | 200.00 |
| 2. | Ritonavir | 25.00 | 50.00 | 25.00 | 50.00 | 25.00 | 50.00 | 25.00 | 50.00 |
| 3. | Copovidone | 295.85 | 591.70 | 295.85 | 591.70 | 295.85 | 591.70 | 295.85 | 591.70 |
| 4. | Sorbitan Monolaurate | 41.50 | 83.00 | 41.50 | 83.00 | 41.50 | 83.00 | 41.50 | 83.00 |
| 5. | Colloidal Silicon Dioxide | 6.10 | 12.20 | 6.10 | 12.20 | 6.10 | 12.20 | 6.10 | 12.20 |
| 6. | Polyethylene Glycol (PEG 6000) | - | - | 60.00 | 120.00 | - | - | 60.00 | 120.00 |

| **Part (b)** | | | | | | | | | |
|---|---|---|---|---|---|---|---|---|---|
| 7. | Lactose Monohydrate | 181.50 | 363.00 | 121.50 | 243.00 | 151.50 | 303.00 | 91.50 | 183.00 |
| 8. | Colloidal Silicon Dioxide | 4.05 | 8.10 | 4.05 | 8.10 | 4.05 | 8.10 | 4.05 | 8.1 |
| 9. | Sodium Stearyl Fumarate | 6.00 | 12.00 | 6.00 | 12.00 | 6.00 | 12.00 | 6.00 | 12.00 |
| **Total Weight of Uncoated Tablet** | | **660.00** | **1320.00** | **660.00** | **1320.00** | **630.00** | **1260.00** | **630.00** | **1260.00** |
| 10. | Opadry® Yellow 16C82767 | 19.80 | 39.60 | 19.80 | 39.60 | 18.90 | 37.80 | 18.90 | 37.80 |
| 11. | Purified Water | q.s. | q.s. | q.s. | q.s. | q.s. | q.s. | q.s. | q.s. |
| **Total Weight of Coated Tablet** | | **679.80** | **1359.60** | **679.80** | **1359.60** | **648.90** | **1297.80** | **648.90** | **1297.80** |

### General Procedure for Preparation of Examples 6 - 13

### Part (a):

The effective amounts of ritonavir and lopinavir were blended with colloidal silicon dioxide and either copovidone (for Examples 6, Example 7, Example 10 and Example 11) or a combination of copovidone and polyethylene glycol (for Example 8, Example 9, Example 12 and Example 13) in a suitable mixer. To the blend, sorbitan monolaurate was added and further blended. The blend obtained was melted and the molten mass was subjected to extrusion at a suitable temperature in a suitable Melt Extruder. The molten extrudate mass was then cooled and suitably sized to obtain a solid dispersion.

### Part (b):

The excipients of Part (b), lactose monohydrate and colloidal silicon dioxide, were blended and mixed with the dispersion of Part (a) in a blender. To the homogeneous blend obtained sodium stearyl fumarate was added and blended.

The final blend was compressed to tablets using appropriate tooling. The tablets were then film-coated using Opadry® Yellow 16C82767 aqueous suspension to a target weight build up of approximately 2.5% w/w.

The tablets of Example 11 and Example 13 were subjected to in-vitro dissolution studies in a USP Type II Apparatus, at 75rpm, at a temperature of 37°C±0.5°C in 900mL using two different media: (a) aqueous medium having 0.06M POEIOLE (Polyoxyethylene 10 lauryl ether) and (b) phosphate buffer 6.8 and 0.06M POEIOLE. Aliquot of sample was withdrawn at predetermined time intervals from media (a) and analysed for lopinavir, and the sample withdrawn from media (b) was analysed for ritonavir. Dissolution profiles of these tablets are provided in Table 1.

**Table 1: In-vitro release pattern of lopinavir/ritonavir from the tablets prepared as per compositions in Example 11 and Example 13 in USP II Apparatus in 900mL of (a) aqueous medium having 0.06M POEIOLE (for lopinavir) and (b) phosphate buffer 6.8 and 0.06M POEIOLE (for ritonavir), at 75 rpm at a temperature of 37°C±0.5°C.**

| **Time (min)** | **Percent of Drugs Released from the Composition Prepared In** | | | |
|---|---|---|---|---|
| | **Example 11** | | **Example 13** | |
| | **Lopinavir** | **Ritonavir** | **Lopinavir** | **Ritonavir** |
| 0 | 0 | 0 | 0 | 0 |
| 15 | 32 | 32 | 32 | 32 |
| 30 | 60 | 60 | 61 | 61 |
| 45 | 81 | 81 | 81 | 81 |
| 60 | 94 | 94 | 93 | 91 |
| 90 | 100 | 100 | 100 | 101 |
| 120 | 100 | 100 | 100 | 101 |

## Claims

1. A solid dosage form comprising a solid dispersion composition, which comprises:
(a) at least one HIV protease inhibitor;
(b) a water soluble polymer having a glass transition temperature (Tg) of at least about 50 °C in an amount of less than 50% by weight of the solid dosage form;
(c) a pharmaceutically acceptable surfactant;
(d) optionally, a pharmaceutically acceptable carrier; and
(e) optionally, one or more pharmaceutically acceptable excipients.

2. The solid dosage form according to claim 1, wherein the HIV protease inhibitor(s) comprises ritonavir, lopinavir, atazanavir, amprenavir, fosamprenavir, saquinavir, tipranavir, or darunavir, or combinations thereof.

3. The solid dosage form according to claim 2, wherein the HIV protease inhibitor comprises ritonavir.

4. The solid dosage form according to claim 2, wherein the HIV protease inhibitors comprise ritonavir and lopinavir.

5. The solid dosage form according to claim 1, wherein the solid dispersion comprises two HIV protease inhibitors in a ratio of about 1:15 to about 15:1 by weight.

6. The solid dosage form according to claim 4, wherein lopinavir and ritonavir are in a ratio of 1:4.

7. The solid dosage form according to claim 1, wherein the water soluble polymer is present in an amount from about 35% to about 48% by weight of the solid dosage form.

8. The solid dosage form according to claim 1, wherein the water soluble polymer comprises copolymer of N-vinyl pyrrolidone and vinyl acetate.

9. The solid dosage form according to claim 1, wherein the pharmaceutically acceptable surfactant comprises polyoxyethylene castor oil derivates, sorbitan monolaurate, stearoyl macrogolglycerides or combinations thereof.

10. The solid dosage form according to claim 1, wherein the pharmaceutically acceptable surfactant is present in an amount of about 1% to about 20% by weight of the solid dosage form.

11. The solid dosage form according to claim 1, wherein the pharmaceutically acceptable carrier has a glass transition temperature (Tg) of less than 50°C.

12. The solid dosage form according to claim 11, wherein the pharmaceutically acceptable carrier comprises polyethylene glycol.

13. The solid dosage form according to claim 12, wherein the pharmaceutically acceptable carrier comprises polyethylene glycol 6000.

14. The solid dosage form according to claim 1, wherein the pharmaceutically acceptable carrier is present in an amount from 0% to about 30% by weight of the solid dosage form.

15. A process of preparation of the solid dosage form according to claim 1, wherein the process comprises of the following steps:
(a) blending at least one HIV protease inhibitor with a water soluble polymer having a glass transition temperature (Tg) of at least about 50°C, optionally, a pharmaceutically acceptable carrier and optionally, one or more pharmaceutically acceptable excipients in a suitable mixer;
(b) mixing the blend of step (a) with the pharmaceutically acceptable surfactant and further blending;
(c) transferring the blend of step (b) to a suitable melt-extruder and melting at appropriate temperature to form a molten extrudate mass;
(d) cooling the molten extrudate mass of step (c), and sizing to obtain a solid dispersion;
(e) blending the solid dispersion of step (d) with one or more pharmaceutically acceptable excipient(s) in a suitable blender to obtain the final blend;
(f) processing the final blend of step (e) into a solid dosage form using appropriate tooling.
